# EUROPEAN PATENT APPLICATION

(11) **EP 1 396 537 A1**
(43) Date of publication of application: **10.03.2004**
(21) Application number: 02292177.9
(22) Date of filing: 04.09.2002
(51) Int. Cl.: C12N 9/88, A61K 38/51, A61P 3/00

(54) **Treatment of elevated plasma homocysteine**

(71) Applicant: Avidis SA, 63360 Saint Beauzire (FR)
(72) Inventor: The designation of the inventor has not yet been filed
(74) Representative: Brasnett, Adrian Hugh

(57) **Abstract**

The present invention provides method of treatment of elevated plasma homocysteine in a human or animal subject which method comprises administering to the subject an effective amount of a truncated cystathionine beta-synthase (CBS). The fragment may be one which lacks haem as a co-factor, does not require S-adenosyl methionine for activation while being active in human plasma. Such fragments include amino acids 40-413 of SEQ ID NO:2 and a novel fragment thereof being amino acids 73-414 of SEQ ID NO:2.

SEQUENCE LISTING

<110> AVIDIS SA

<120> Treatment of Elevated Plasma Homocysteine

<130> AHB/FP6091078

<140> EP 02292177.9
<141> 2002-09-04

<160> 4

<170> PatentIn Ver. 2.1

<210> 1
<211> 1924
<212> DNA
<213> Homo sapiens

<220>
<221> CDS
<222> (181)..(1836)

<400> 1
tgcagggcca ggacgcacgt ttcaagctca tcagtaaagg ttccttaaat tcccgaagca 60
agaagttaac caagtaaaac agcatcggaa caccaggatc ccatgacaga ttctgttgtc 120
acgtctcctt acagagtttg agcggtgctg aactgtcagc accatctgtc cggtcccagc 180
atg cct tct gag acc ccc cag gca gaa gtg ggg ccc aca ggc tgc ccc 228
cac cgc tca ggg cca cac tcg gcg aag ggg agc ctg gag aag ggg tcc 276
cca gag gat aag gaa gcc aag gag ccc ctg tgg atc cgg ccc gat gct    324
ccg agc agg tgc acc tgg cag ctg ggc cgg cct gcc tcc gag tcc cca    372
cat cac cac act gcc ccg gca aaa tct cca aaa atc ttg cca gat att    420
ctg aag aaa atc ggg gac acc cct atg gtc aga atc aac aag att ggg    468
aag aag ttc ggc ctg aag tgt gag ctc ttg gcc aag tgt gag ttc ttc    516
aac gcg ggc ggg agc gtg aag gac cgc atc agc ctg cgg atg att gag    564
gat gct gag cgc gac ggg acg ctg aag ccc ggg gac acg att atc gag    612
ccg aca tcc ggg aac acc ggg atc ggg ctg gcc ctg gct gcg gca gtg    660
agg ggc tat cgc tgc atc atc gtg atg cca gag aag atg agc tcc gag    708
aag gtg gac gtg ctg cgg gca ctg ggg gct gag att gtg agg acg ccc    756
acc aat gcc agg ttc gac tcc ccg gag tca cac gtg ggg gtg gcc tgg    804
cgg ctg aag aac gaa atc ccc aat tct cac atc cta gac cag tac cgc    852
aac gcc agc aac ccc ctg gct cac tac gac acc acc gct gat gag atc    900
ctg cag cag tgt gat ggg aag ctg gac atg ctg gtg gct tca gtg ggc    948
acg ggc ggc acc atc acg ggc att gcc agg aag ctg aag gag aag tgt    996
cct gga tgc agg atc att ggg gtg gat ccc gaa ggg tcc atc ctc gca    1044
gag ccg gag gag ctg aac cag acg gag cag aca acc tac gag gtg gaa    1092
ggg atc ggc tac gac ttc atc ccc acg gtg ctg gac agg acg gtg gtg    1140
gac aag tgg ttc aag agc aac gat gag gag gcg ttc acc ttt gcc cgc 1188
atg ctg atc gcg caa gag ggg ctg ctg tgc ggt ggc agt gct ggc agc 1236
acg gtg gcg gtg gcc gtg aag gct gcg cag gag ctg cag gag ggc cag 1284
cgc tgc gtg gtc att ctg ccc gac tca gtg cgg aac tac atg acc aag 1332
ttc ctg agc gac agg tgg atg ctg cag aag ggc ttt ctg aag gag gag 1380
gac ctc acg gag aag aag ccc tgg tgg tgg cac ctc cgt gtt cag gag 1428
ctg ggc ctg tca gcc ccg ctg acc gtg ctc ccg acc atc acc tgt ggg 1476
cac acc atc gag atc ctc cgg gag aag ggc ttc gac cag gcg ccc gtg 1524
gtg gat gag gcg ggg gta atc ctg gga atg gtg acg ctt ggg aac atg 1572
ctc tcg tcc ctg ctt gcc ggg aag gtg cag ccg tca gac caa gtt ggc    1620
aaa gtc atc tac aag cag ttc aaa cag atc cgc ctc acg gac acg ctg    1668
ggc agg ctc tcg cac atc ctg gag atg gac cac ttc gcc ctg gtg gtg    1716
cac gag cag atc cag tac cac agc acc ggg aag tcc agt cag cgg cag    1764
atg gtg ttc ggg gtg gtc acc gcc att gac ttg ctg aac ttc gtg gcc    1812
gcc cag gag cgg gac cag aag tga agtccggagc gctgggcggt gtggagcggg    1866
cccgccaccc ttgcccactt ctccttcgct ttcctgagcc ctaaacacac gcgtgatt    1924

<210> 2
<211> 551
<212> PRT
<213> Homo sapiens

<400> 2

<210> 3
<211> 1032
<212> DNA
<213> Artificial Sequence

<220>
<223> Description of Artificial Sequence: Truncation
      suitable for expression in Escherichia coli

<220>
<221> CDS
<222> (1)..(1029)

<400> 3
atg agc cca aaa atc ctg ccg gac atc ctg aag aaa att ggt gac acc     48
Met Ser Pro Lys Ile Leu Pro Asp Ile Leu Lys Lys Ile Gly Asp Thr
  1               5                  10                  15

ccg atg gtt cgt atc aac aaa atc ggt aag aaa ttc ggc ctg aaa tgt     96
Pro Met Val Arg Ile Asn Lys Ile Gly Lys Lys Phe Gly Leu Lys Cys
             20                  25                  30

gaa cta ctg gcg aaa tgc gag ttc ttt aac gct ggt ggc tcc gta aaa    144
Glu Leu Leu Ala Lys Cys Glu Phe Phe Asn Ala Gly Gly Ser Val Lys
         35                  40                  45

gac cgt atc tcc ctg cgc atg atc gaa gac gca gaa cgt gat ggt act    192
Asp Arg Ile Ser Leu Arg Met Ile Glu Asp Ala Glu Arg Asp Gly Thr
     50                  55                  60

ctg aaa ccg ggc gac aca att atc gaa ccg acc tct ggt aac act ggc    240
Leu Lys Pro Gly Asp Thr Ile Ile Glu Pro Thr Ser Gly Asn Thr Gly
 65                  70                  75                  80

atc ggt ctg gct ctg gcc gca gcg gtt cgt ggc tac cgc tgt atc atc    288
Ile Gly Leu Ala Leu Ala Ala Ala Val Arg Gly Tyr Arg Cys Ile Ile
                 85                  90                  95

gtt atg cct gaa aag atg agc tcc gag aaa gtc gac gta ctg cgt gct 336
Val Met Pro Glu Lys Met Ser Ser Glu Lys Val Asp Val Leu Arg Ala
            100                 105                 110

ctg ggt gcc gaa atc gtt cgt acc ccg act aac gca cgt ttc gac tct 384
Leu Gly Ala Glu Ile Val Arg Thr Pro Thr Asn Ala Arg Phe Asp Ser
        115                 120                 125

ccg gaa tcc cac gtc ggc gta gcg tgg cgc ctg aaa aac gaa atc cca 432
Pro Glu Ser His Val Gly Val Ala Trp Arg Leu Lys Asn Glu Ile Pro
    130                 135                 140

aat tca cac atc ctg gac cag tac cgt aac gcg tct aac ccg ctg gct 480
Asn Ser His Ile Leu Asp Gln Tyr Arg Asn Ala Ser Asn Pro Leu Ala
145                 150                 155                 160

cat tat gat acc act gca gac gaa atc ctg cag caa tgc gac ggt aaa 528
His Tyr Asp Thr Thr Ala Asp Glu Ile Leu Gln Gln Cys Asp Gly Lys
                165                 170                 175

ctg gat atg tta gtt gcg tcc gtt ggt acc ggc ggt acg att act ggt 576
Leu Asp Met Leu Val Ala Ser Val Gly Thr Gly Gly Thr Ile Thr Gly
            180                 185                 190

atc gct cgt aaa ctg aag gaa aaa tgt ccg ggt tgc cgc atc att ggc 624
Ile Ala Arg Lys Leu Lys Glu Lys Cys Pro Gly Cys Arg Ile Ile Gly
        195                 200                 205

gtt gac ccg gaa ggt agc atc ctg gca gag cct gaa gag ctc aac cag 672
Val Asp Pro Glu Gly Ser Ile Leu Ala Glu Pro Glu Glu Leu Asn Gln
    210                 215                 220

act gaa cag aca acc tac gaa gtt gaa ggt atc ggc tat gac ttc atc 720
Thr Glu Gln Thr Thr Tyr Glu Val Glu Gly Ile Gly Tyr Asp Phe Ile
225                 230                 235                 240

ccg acc gta ctg gat cgt act gtg gtt gac aaa tgg ttc aag tcc aac 768
Pro Thr Val Leu Asp Arg Thr Val Val Asp Lys Trp Phe Lys Ser Asn
                245                 250                 255

gat gaa gag gcg ttc acc ttt gct cgt atg ctg atc gca cag gaa ggt 816
Asp Glu Glu Ala Phe Thr Phe Ala Arg Met Leu Ile Ala Gln Glu Gly
            260                 265                 270

ctg ctt tgt ggc ggt tct gct ggc agc act gtt gcc gtg gca gta aaa 864
Leu Leu Cys Gly Gly Ser Ala Gly Ser Thr Val Ala Val Ala Val Lys
        275                 280                 285

gcg gct cag gaa ctg cag gaa ggt cag cgc tgc gtt gtg atc ctg ccg 912
Ala Ala Gln Glu Leu Gln Glu Gly Gln Arg Cys Val Val Ile Leu Pro
    290                 295                 300

gac tcc gta cgt aac tac atg act aaa ttc ctg tct gat cgt tgg atg 960
Asp Ser Val Arg Asn Tyr Met Thr Lys Phe Leu Ser Asp Arg Trp Met
305                 310                 315                 320

ctg cag aaa ggc ttc ctc aag gaa gag gac ctg act gaa aaa aaa ccg 1008
Leu Gln Lys Gly Phe Leu Lys Glu Glu Asp Leu Thr Glu Lys Lys Pro
                325                 330                 335

tgg tgg tgg cac ctg cgt taa taa 1032
Trp Trp Trp His Leu Arg
            340

<210> 4

<211> 342

<212> PRT

<213> Artificial Sequence

<220>

<223> Description of Artificial Sequence: Truncation
      suitable for expression in Escherichia coli

<400> 4

Met Ser Pro Lys Ile Leu Pro Asp Ile Leu Lys Lys Ile Gly Asp Thr
  1               5                  10                  15

Pro Met Val Arg Ile Asn Lys Ile Gly Lys Lys Phe Gly Leu Lys Cys
             20                  25                  30

Glu Leu Leu Ala Lys Cys Glu Phe Phe Asn Ala Gly Gly Ser Val Lys
         35                  40                  45

Asp Arg Ile Ser Leu Arg Met Ile Glu Asp Ala Glu Arg Asp Gly Thr
     50                  55                  60

Leu Lys Pro Gly Asp Thr Ile Ile Glu Pro Thr Ser Gly Asn Thr Gly
 65                  70                  75                  80

Ile Gly Leu Ala Leu Ala Ala Ala Val Arg Gly Tyr Arg Cys Ile Ile
                 85                  90                  95

Val Met Pro Glu Lys Met Ser Ser Glu Lys Val Asp Val Leu Arg Ala
            100                 105                 110

Leu Gly Ala Glu Ile Val Arg Thr Pro Thr Asn Ala Arg Phe Asp Ser
        115                 120                 125

Pro Glu Ser His Val Gly Val Ala Trp Arg Leu Lys Asn Glu Ile Pro
    130                 135                 140

Asn Ser His Ile Leu Asp Gln Tyr Arg Asn Ala Ser Asn Pro Leu Ala
145                 150                 155                 160

His Tyr Asp Thr Thr Ala Asp Glu Ile Leu Gln Gln Cys Asp Gly Lys
                165                 170                 175

Leu Asp Met Leu Val Ala Ser Val Gly Thr Gly Gly Thr Ile Thr Gly
            180                 185                 190

Ile Ala Arg Lys Leu Lys Glu Lys Cys Pro Gly Cys Arg Ile Ile Gly
        195                 200                 205

Val Asp Pro Glu Gly Ser Ile Leu Ala Glu Pro Glu Glu Leu Asn Gln
    210                 215                 220

Thr Glu Gln Thr Thr Tyr Glu Val Glu Gly Ile Gly Tyr Asp Phe Ile
225                 230                 235                 240
Pro Thr Val Leu Asp Arg Thr Val Val Asp Lys Trp Phe Lys Ser Asn
                245                 250                 255
Asp Glu Glu Ala Phe Thr Phe Ala Arg Met Leu Ile Ala Gln Glu Gly
            260                 265                 270
Leu Leu Cys Gly Gly Ser Ala Gly Ser Thr Val Ala Val Ala Val Lys
        275                 280                 285
Ala Ala Gln Glu Leu Gln Glu Gly Gln Arg Cys Val Val Ile Leu Pro
    290                 295                 300
Asp Ser Val Arg Asn Tyr Met Thr Lys Phe Leu Ser Asp Arg Trp Met
305                 310                 315                 320
Leu Gln Lys Gly Phe Leu Lys Glu Glu Asp Leu Thr Glu Lys Lys Pro
                325                 330                 335
Trp Trp Trp His Leu Arg
            340

## Description

### Field of the Invention

The present invention relates to methods of treatment of elevated levels of plasma homocysteine in mammals, particularly humans.

### Background to the Invention

Elevated plasma homocysteine is a risk factor for a number of serious human diseases, particularly those of the cardiovascular and central nervous systems, but also of several other systems.

The administration of vitamins such as vitamin B6 or folic acid can lower plasma homocysteine in a significant number of patients, but not in all. In humans, an enzyme is known which is capable of decreasing plasma homocysteine by coupling it to serine, thus creating cystathionine. Cystathionine is more easily metabolised and excreted from the body than homocysteine.

This enzyme, called cystathionine beta-synthase, has been cloned, as described in US Patent 5,523,225 "DNA sequence encoding human cystathionine beta-synthase", and recombinant versions have since been produced in the bacterium *Escherichia coli.* The same patent proposes the use of the enzyme as a method of reducing plasma homocysteine.

A modified version of human cystathionine beta-synthase has been described in "Structure of human cystathionine beta-synthase: a unique pyridoxal 5'-phosphate-dependent heme protein" by M. Meier, M. Janosik, V. Kery, J.P. Kraus and P. Burkhard in EMBO Journal (2001) Volume 20, pages 3910-3916. This version of cystathionine beta-synthase comprises amino acid residues 1-413, that is with a truncation of the C-terminal amino acids 414-551 was made to facilitate crystallisation of the "active core" of the enzyme. The citation also describes amino acid residues 40-413 as an "active core" of the enzyme which forms dimers, as opposed to the tetramers observed with the native enzyme.

### Disclosure of the Invention

The present invention provides a method of treatment of elevated plasma homocysteine in a human or animal subject which method comprises administering to the subject an effective amount of a truncated cystathionine beta-synthase (CBS).

In one aspect, said truncated CBS can be characterised by the following properties:
i) lacking haem as a co-factor;
ii) not requiring S-adenosyl methionine for activation; and
iii) being active in human plasma.

Alternatively, the truncated CBS may be represented by amino acids 40-413 of SEQ ID NO:2, or a fragment thereof retaining at least amino acids 73-401 of SEQ ID NO:2.

The present invention further provides a CBS as defined above for use in a method of treatment of elevated plasma homocysteine in a human or animal subject.

The treatment of the invention is useful for prevention of the deleterious effects of elevated plasma homocysteine.

Deleterious effects of elevated plasma homocysteine are found particularly in human subjects with a mutation in their CBS gene.

### Description of the Sequence listing.

SEQ ID NOs:1 and 2 shows the full length human CBS cDNA and sequence and translation thereof.

SEQ ID NOs:3 and 4 shows the DNA sequence and translation thereof of a preferred truncation of invention suitable for expression in *Escherichia coli.*

### Detailed Description of the Invention.

Native CBS, whether produced recombinantly or isolated from a natural source, has significant disadvantages for the treatment of homocysteinemia. These disadvantages are: firstly, the manufacture of the enzyme is difficult because the enzyme has haem as a co-factor, secondly the full-length enzyme has a strong tendency to aggregate and thirdly the enzyme is activated by the co-factor S-adenosyl methionine. Thus the efficacious production of the enzyme in a form that will be active in human plasma is prevented by the requirement for these two co-factors and the strong tendency to aggregate.

In the present invention, the engineered versions of the human enzyme provided have the advantage of not requiring either of these co-factors and have a significantly reduced tendency to aggregate but which are nevertheless active in human plasma, and are easier to manufacture than the native enzyme.

The engineered versions of the enzyme are smaller in size than the native enzyme because of two deletions. An N-terminal deletion which is of approximately seventy amino acids deletes the haem binding domain of the protein, without abolishing the enzyme's activity. A second deletion from the C-terminus of at least one hundred and thirty residues deletes the S-adenosyl methionine binding domain without abolishing the activity of the enzyme whether the haem binding domain is present or absent. Thus the preferred versions of the enzyme for administration to humans have deletions at both the N-terminus and at the C-terminus. The advantages for manufacture or for use as a human therapeutic of the truncated versions of human cystathionine beta-synthase have not been suggested in the art.

### Definitions

By "elevated plasma homocysteine" it is meant a level of this compound in the plasma of a subject which leads to one or more symptoms of homocystinuria (Mudd et al., The Metabolic Basis of Inherited Disease, 6th Ed. McGraw-Hill, New York, pp. 693-734 (1989)). Untreated patients develop a number of phenotypic traits which include skeletal abnormalities, dislocated optic lenses, mild to profound mental retardation, and vascular disorders (Mudd et al., *ibid*). Some patients respond to vitamin B6 administration while others are unresponsive to this therapeutic intervention (Mudd et al., *ibi*d; and, Lipson et al., J. Clin. Invest., 66, 188-193 (1980)). A growing body of evidence now suggests that vascular disorders found in one-third of the patients with premature arterial disease or cerebrovascular disease are the result of mild hyperhomocysteinemia some of which may be due to heterozygous CBS deficiency (Boers *et al.,* N. Engl. J. Med., 313, 709715 (1985); and, Clarke *et al.,* N. Engl. J. Med., 324, 1149-1155 (1991)).

Diagnosis may be by the clinical and biochemical criteria discussed above, or may be by genotyping of the CBS genes of the subject to identify mutations. The wild type cDNA sequence is provided herein as SEQ ID NO:1.

Reference to an "animal" subject includes in particular mammals which may be used experimentally as models of homocystinuria. For example, knock-out animals may be developed for the provision of clinical testing. However, the use of the present invention in the treatment of a human subject is preferred.

By "truncated cystathionine beta-synthase" it is meant any form of this protein which is shorter than the wild type human protein or its animal, particularly mammalian, homologues, and preferably which has the properties (i) to (iii) as further defined above.

Preferably, the protein comprises both an N-terminal and C-terminal truncation. For example, the protein may consist of residues 40-413 of SEQ ID NO:2 or a fragment thereof. The fragment desirably includes at least residues 73-401. One such fragment, corresponding to 73-413 of SEQ ID NO:2, is illustrated as SEQ ID NO:4 (residues 2 to 342 of SEQ ID NO:4), which additionally includes a N-terminal methionine for the purposes of recombinant expression. Fragments of CBS consisting of residues 73-413 of SEQ ID NO:2, or fragments thereof including at least residues 73-401 of SEQ ID NO:2, form a further aspect of the invention, as do compositions comprising these fragments.

The proteins of the invention may be in substantially isolated form. The proteins may of course be formulated with diluents or adjuvants and still for practical purposes be isolated. The proteins may be glycosylated, either naturally or by systems of heterologous eukaryotic cells, or they may be (for example if produced by expression in a prokaryotic cell) unglycosylated. Protiens may be phosphorylated and/or acetylated.

A protein of the invention may also be in a substantially purified form, in which case it will generally comprise the protein in a preparation in which more than 90%, e.g. 95%, 98% or 99% of the protein in the preparation is a protein of the invention.

By "lacking haem as a co-factor" it is meant that the protein is active *in vitro* in human plasma without the need for addition of haem co-factor.

By "not requiring S-adenosyl methionine for activation" it is meant that the protein is active *in vitro* in human plasma without the need for addition of S-adenosyl methionine.

By "being active in human plasma" it is meant that the protein is capable of producing cystathionine from serine and homocysteine in the presence of pyridoxal 5' phosphate. This may be ascertained in a sample of human plasma *in vitro* or *in vivo*. Assays for CBS activity are well known to those skilled in the art and can be found in Kraus, Methods Enzymol 143:388-394 (1987)], and in US patent 5,656,425.

By "treatment" it will be understood that this refers to any administration of a polypeptide intended to alleviate the severity of a disease being treated, to provide relief from the symptoms of the disease or to prevent or slow down the development of the disease in an individual with a disease condition or at risk of developing the disease condition.

### Compositions

Proteins for use in accordance with the invention may be formulated as a pharmaceutical composition comprising diluents such as water, saline, dextrose, glycerol, ethanol and the like. Compositions may be formulated for injection, for example for intravenous injection. The composition to be administered may also auxiliary substances such as pH buffering agents and the like. Actual methods of preparing such dosage forms are known, or will be apparent, to those skilled in this art; for example, see Remington's Pharmaceutical Sciences, Mack Publishing Company, Easton, Pennsylvania, 19th Edition, 1995.

Suitable doses of polypeptides will ultimately be at the discretion of the physician taking account of the nature of the condition to be treated and the condition of the patient.

### Production of Proteins

Proteins for use in accordance with the invention may be made using recombinant DNA technology in accordance with routine procedures known in the art. In general, a nucleic acid vector, generally DNA or RNA, which encodes a truncated CBS protein for use in the invention may be provided which contains coding sequence for CBS operably linked to a promoter suitable for expression of the protein in a desired host cell.

The vectors may be provided with an origin of replication and optionally a regulator of the promoter. The vectors may contain one or more selectable marker genes. There are a wide variety of prokaryotic and eukaryotic expression vectors known as such in the art, and the present invention may utilise any vector according to the individual preferences of those of skill in the art.

The vectors encoding the novel proteins of the invention form a further aspect of the present invention. In one aspect, the vector is a prokaryotic vector, particularly an E. coli vector which carries SEQ ID NO:3 or a part thereof. This sequence has been adapted from the wild-type CBS-encoding sequence for improved expression in *E. coli.*

A wide variety of prokaryotic host cells can be used in the method of the present invention. These hosts may include strains of Escherichia, Pseudomonas, Bacillus, Lactobacillus, Thermophilus, Salmonella, Enterobacteriacae or Streptomyces.

Prokaryotic vectors includes vectors bacterial plasmids, e.g., plasmids derived from E. coli including ColEI, pCR1, pBR322, pMB9 and their derivatives, wider host range plasmids, e.g., RP4; phage DNAs, e.g., the numerous derivatives of phage A, e.g., NM989, and other DNA phages, e.g., M13 and filamentous single stranded DNA phages.

The promoter may be an inducible promoter. Suitable promoters include the T7 promoter, the tac promoter, the trp promoter, the lambda promoters P_{L} or P_{R} and others well known to those skilled in the art.

A wide variety of eukaryotic host cells may also be used, including for example yeast, insect and mammalian cells. Mammalian cells include CHO and mouse cells, African green monkey cells, such as COS-1, and human cells.

Many eukaryotic vectors suitable for expression of proteins are known. These vectors may be designed to be chromosomally incorporated into a eukaryotic cell genome or to be maintained extrachromosomally, or to be maintained only transiently in eukaryotic cells. The nucleic acid may be operably linked to a suitable promoter, such as a strong viral promoter including a CMV promoter, and SV40 T-antigen promoter or a retroviral LTR.

To obtain a product of the invention, host cells carrying a vector of the invention may be cultured under conditions suitable for expression of the protein, and the protein recovered from the cells or from the culture medium.

Host cells comprising a novel vector of the invention, and their use, form a further aspect of the present invention.

### Example

A nucleic acid sequence (SEQ ID NO:3) which encodes an engineered version of human cystathionine beta-synthase (SEQ ID NO:4) was prepared, and cloned in a bacterial expression vector. The resulting plasmid is used to produce by standard methods a novel protein, which forms a further aspect of the present invention, in significant quantities and which has cystathionine beta-synthase activity, but no requirement for either haem or S-adenosyl methionine as co-factors, or cleavage by a protease to release the desired protein fragment. The protein, when produced in *E. coli,* may be recovered from the cells by standard purification methods. When recovered, some or all of the protein recovered may lack the N-terminal methionine due to its removal by bacterial cellular processes.

This engineered version of human cystathionine beta-synthase can prepared as a novel pharmaceutical composition and may be administered in a variety of formulations to humans to treat or prevent the effects of raised plasma homocysteine.

SEQUENCE LISTING

SEQ ID NO:1 (DNA) & SEQ ID NO:2 (Protein)
TGCAGGGCCAGGACGCCACGTTTCAAGCTCATCAGTAAAGGTTCCTTAAATTCCCGAAGCA 60
AGAAGTTAACCAAGTAAAA CAGCATCGGAACACCAGGATCCCATGACAGATTCTGTTGTC 120
ACGTCTCCTTACAGAGTTTGAGCGGTGCTGAACTGTCAGCACCATCTGTCCGGTCCCAGC 180
ATGCCTTCTGAGACCCCCCAGGCAGAAGTGGGGCCCACAGGCTGCCCC 228

1 5 10 15

CACCGCTCAGGGCCACACTCGGCGAAGGGGAGCCTGGAGAAGGGGTCC 276

20 25 30

CCAGAGGATAAGGAAGCCAAGGAGCCCCTGTGGATCCGGCCCGATGCT 324

35 40 45

CCGAGCAGGTGCACCTGGCAGCTGGGCCGGCCTGCCTCCGAGTCCCCA 372

50 55 60

CATCACCACACTGCCCCGGCAAAATCTCCAAAAATCTTGCCAGATATT 420

65 70 75 80

CTGAAGAAAATCGGGGACACCCCTATGGTCAGAATCAACAAGATTGGG 468

85 90 95

AAGAAGTTCGGCCTGAAGTGTGAGCTCTTGGCCAAGTGTGAGTTCTTC 516

100 105 110

AACGCGGGCGGGAGCGTGAAGGACCGCATCAGCCTGCGGATGATTGAG 564

115 120 125

GATGCTGAGCGCGACGGGACGCTGAAGCCCGGGGACACGATTATCGAG 612

130 135 140

CCGACATCCGGGAACACCGGGATCGGGCTGGCCCTGGCTGCGGCAGTG 660

145 150 155 160

AGGGGCTATCGCTGCATCATCGTGATGCCAGAGAAGATGAGCTCCGAG 708

165 170 175

AAGGTGGACGTGCTGCGGGCACTGGGGGCTGAGATTGTGAGGACGCCC 756

180 185 190

ACCAATGCCAGGTTCGACTCCCCGGAGTCACACGTGGGGGTGGCCTGG 804

195 200 205

CGGCTGAAGAACGAAATCCCCAATTCTCACATCCTAGACCAGTACCGC 852

210 215 220

AACGCCAGCAACCCCCTGGCTCACTACGACACCACCGCTGATGAGATC 900

225 230 235 240

CTGCAGCAGTGTGATGGGAAGCTGGACATGCTGGTGGCTTCAGTGGGC 948


245 250 255

ACGGGCGGCACCATCACGGGCATTGCCAGGAAGCTGAAGGAGAAGTGT 996

260 265 270

CCTGGATGCAGGATCATTGGGGTGGATCCCGAAGGGTCCATCCTCGCA 1044

275 280 285

GAGCCGGAGGAGCTGAACCAGACGGAGCAGACAACCTACGAGGTGGAA 1092

290 295 300

GGGATCGGCTACGACTTCATCCCCACGGTGCTGGACAGGACGGTGGTG 1140

305 310 315 320

GACAAGTGGTTCAAGAGCAACGATGAGGAGGCGTTCACCTTTGCCCGC 1188

325 330 335

ATGCTGATCGCGCAAGAGGGGCTGCTGTGCGGTGGCAGTGCTGGCAGC 1236

340 345 350

ACGGTGGCGGTGGCCGTGAAGGCTGCGCAGGAGCTGCAGGAGGGCCAG 1284

355 360 365

CGCTGCGTGGTCATTCTGCCCGACTCAGTGCGGAACTACATGACCAAG 1332

370 375 380

TTCCTGAGCGACAGGTGGATGCTGCAGAAGGGCTTTCTGAAGGAGGAG 1380

385 390 395 400

GACCTCACGGAGAAGAAGCCCTGGTGGTGGCACCTCCGTGTTCAGGAG 1428

405 410 415

CTGGGCCTGTCAGCCCCGCTGACCGTGCTCCCGACCATCACCTGTGGG 1476

420 425 430

CACACCATCGAGATCCTCCGGGAGAAGGGCTTCGACCAGGCGCCCGTG 1524

435 440 445
GTGGATGAGGCGGGGGTAATCCTGGGAATGGTGACGCTTGGGAACATG 1572

450 455 460
CTCTCGTCCCTGCTTGCCGGGAAGGTGCAGCCGTCAGACCAAGTTGGC 1620

465 470 475 480
AAAGTCATCTACAAGCAGTTCAAACAGATCCGCCTCACGGACACGCTG 1668

485 490 495
GGCAGGCTCTCGCACATCCTGGAGATGGACCACTTCGCCCTGGTGGTG 1716

500 505 510
CACGAGCAGATCCAGTACCACAGCACCGGGAAGTCCAGTCAGCGGCAG 1764

515 520 525
ATGGTGTTCGGGGTGGTCACCGCCATTGACTTGCTGAACTTCGTGGCC 1812

530 535 540

GCCCAGGAGCGGGACCAGAAGTGAAGTCCGGAGCGCTGGGCGGTGTGGAGCG          1864

545           550
GGCCCGCCACCCTTGCCCACTTCTCCTTCGCTTTCCTGAGCCCTAAACACACGCGTGATT  1924

SEQ ID NO:3 (DNA) and SEQ ID NO:4 (Protein)

ATG AGC CCA AAA ATC CTG CCG GAC ATC CTG AAG AAA ATT GGT GAC ACC CCG ATG GTT CGT    60
Met Ser Pro Lys Ile Leu Pro Asp Ile Leu Lys Lys Ile Gly Asp Thr Pro Met Val Arg    20

ATC AAC AAA ATC GGT AAG AAA TTC GGC CTG AAA TGT GAA CTA CTG GCG AAA TGC GAG TTC   120
Ile Asn Lys Ile Gly Lys Lys Phe Gly Leu Lys Cys Glu Leu Leu Ala Lys Cys Glu Phe    40

TTT AAC GCT GGT GGC TCC GTA AAA GAC CGT ATC TCC CTG CGC ATG ATC GAA GAC GCA GAA   180
Phe Asn Ala Gly Gly Ser Val Lys Asp Arg Ile Ser Leu Arg Met Ile Glu Asp Ala Glu    60

CGT GAT GGT ACT CTG AAA CCG GGC GAC ACA ATT ATC GAA CCG ACC TCT GGT AAC ACT GGC   240
Arg Asp Gly Thr Leu Lys Pro Gly Asp Thr Ile Ile Glu Pro Thr Ser Gly Asn Thr Gly    80

ATC GGT CTG GCT CTG GCC GCA GCG GTT CGT GGC TAC CGC TGT ATC ATC GTT ATG CCT GAA   300
Ile Gly Leu Ala Leu Ala Ala Ala Val Arg Gly Tyr Arg Cys Ile Ile Val Met Pro Glu   100

AAG ATG AGC TCC GAG AAA GTC GAC GTA CTG CGT GCT CTG GGT GCC GAA ATC GTT CGT ACC   360
Lys Met Ser Ser Glu Lys Val Asp Val Leu Arg Ala Leu Gly Ala Glu Ile Val Arg Thr   120

CCG ACT AAC GCA CGT TTC GAC TCT CCG GAA TCC CAC GTC GGC GTA GCG TGG CGC CTG AAA   420
Pro Thr Asn Ala Arg Phe Asp Ser Pro Glu Ser His Val Gly Val Ala Trp Arg Leu Lys   140

AAC GAA ATC CCA AAT TCA CAC ATC CTG GAC CAG TAC CGT AAC GCG TCT AAC CCG CTG GCT   480
Asn Glu Ile Pro Asn Ser His Ile Leu Asp Gln Tyr Arg Asn Ala Ser Asn Pro Leu Ala   160

CAT TAT GAT ACC ACT GCA GAC GAA ATC CTG CAG CAA TGC GAC GGT AAA CTG GAT ATG TTA   540
His Tyr Asp Thr Thr Ala Asp Glu Ile Leu Gln Gln Cys Asp Gly Lys Leu Asp Met Leu   180

GTT GCG TCC GTT GGT ACC GGC GGT ACG ATT ACT GGT ATC GCT CGT AAA CTG AAG GAA AAA   600
Val Ala Ser Val Gly Thr Gly Gly Thr Ile Thr Gly Ile Ala Arg Lys Leu Lys Glu Lys   200

TGT CCG GGT TGC CGC ATC ATT GGC GTT GAC CCG GAA GGT AGC ATC CTG GCA GAG CCT GAA   660
Cys Pro Gly Cys Arg Ile Ile Gly Val Asp Pro Glu Gly Ser Ile Leu Ala Glu Pro Glu   220

GAG CTC AAC CAG ACT GAA CAG ACA ACC TAC GAA GTT GAA GGT ATC GGC TAT GAC TTC ATC   720
Glu Leu Asn Gln Thr Glu Gln Thr Thr Tyr Glu Val Glu Gly Ile Gly Tyr Asp Phe Ile   240

CCG ACC GTA CTG GAT CGT ACT GTG GTT GAC AAA TGG TTC AAG TCC AAC GAT GAA GAG GCG   780
Pro Thr Val Leu Asp Arg Thr Val Val Asp Lys Trp Phe Lys Ser Asn Asp Glu Glu Ala   260

TTC ACC TTT GCT CGT ATG CTG ATC GCA CAG GAA GGT CTG CTT TGT GGC GGT TCT GCT GGC   840
Phe Thr Phe Ala Arg Met Leu Ile Ala Gln Glu Gly Leu Leu Cys Gly Gly Ser Ala Gly   280

AGC ACT GTT GCC GTG GCA GTA AAA GCG GCT CAG GAA CTG CAG GAA GGT CAG CGC TGC GTT   900
Ser Thr Val Ala Val Ala Val Lys Ala Ala Gln Glu Leu Gln Glu Gly Gln Arg Cys Val   300

GTG ATC CTG CCG GAC TCC GTA CGT AAC TAC ATG ACT AAA TTC CTG TCT GAT CGT TGG ATG   960
Val Ile Leu Pro Asp Ser Val Arg Asn Tyr Met Thr Lys Phe Leu Ser Asp Arg Trp Met   320

CTG CAG AAA GGC TTC CTC AAG GAA GAG GAC CTG ACT GAA AAA AAA CCG TGG TGG TGG CAC  1020
Leu Gln Lys Gly Phe Leu Lys Glu Glu Asp Leu Thr Glu Lys Lys Pro Trp Trp Trp His   340

CTG CGT taa taa                                                                  1032
Leu Arg *   *                                                                     342

## Claims

1. A truncated cystathionine beta-synthase (CBS) for use in a method of treatment of elevated plasma homocysteine in a human or animal subject.

2. A truncated CBS for use according to claim 1 wherein said truncated CBS is **characterised by** the following properties:
i) lacking haem as a co-factor;
ii) not requiring S-adenosyl methionine for activation; and
iii) being active in human plasma.

3. A truncated CBS for use according to claim 1 wherein the truncated CBS is represented by amino acids 40-413 of SEQ ID NO:2 or a fragment thereof retaining at least amino acids 73-401 of SEQ ID NO:2.

4. A protein consisting of SEQ ID NO:4, or a fragment thereof comprising residues 2 to 330 of SEQ ID NO:4.

5. A composition comprising the protein of claim 4, optionally lacking its N-terminal methionine, together with a pharmaceutically acceptable carrier.

6. A recombinant vector comprising the sequence of SEQ ID NO:3 operably linked to a promoter.

7. The vector of claim 6 wherein said promoter is functional in an *E. coli* host cell.

8. A method of treatment of elevated plasma homocysteine in a human or animal subject which method comprises administering to the subject an effective amount of a truncated cystathionine beta-synthase (CBS).

9. The method of claim 2 wherein said truncated CBS is **characterised by** the following properties:
i) lacking haem as a co-factor;
ii) not requiring S-adenosyl methionine for activation; and
iii) being active in human plasma.

10. The method of claim 8 wherein the truncated CBS is represented by amino acids 40-413 of SEQ ID NO:2 or a fragment thereof retaining at least amino acids 73-401 of SEQ ID NO:2.
